# FASCICULE DE BREVET EUROPEEN

(11) **EP 1 863 109 B1**
(45) Date de publication et mention de la délivrance du brevet: **07.09.2016**
(21) Numéro de dépôt: 07014876.2
(22) Date de dépôt: 10.09.1998
(51) Int. Cl.: C07C 311/48, H01G 9/022, H01G 11/56, H01M 10/052, H01M 10/0564, H01M 10/0565, H01M 10/0568, H01M 10/0569, C07C 381/10, H01G 11/62, H01M 6/16, H01M 6/18

(54) **Solvants et nouvelles compositions électrolytiques possédant un large domaine de stabilité et une conductivité élevée**
Solvents and new electrolytic compositions possessing a wide stability domain and high conductivity
Solvents and new electrolytic compositions possessing a wide stability domain and high conductivity

(30) Priorité: 11.09.1997 CA 2215849
(43) Date de publication de la demande: 05.12.2007
(62) Demande divisionnaire de: 98402242.6
(73) Titulaire: ACEP Inc., Montréal, Quebec H2Z 1A4 (CA); UNIVERSITE DE MONTREAL, Montréal, Québec H3C 3J7 (CA); Centre National de la Recherche Scientifique, 75016 Paris (FR)
(72) Inventeur: Armand, Michel, Montréal Québec H3T1N2 (CA); Michot, Christophe, Montréal Québec (CA); Brouillette, Dany, Montréal Québec H3K 1R7 (CA); Baril, Daniel, Montréal Québec H2G 2Y3 (CA); Bergeron, Jean-Yves, Québec H4G 2H1 (CA)
(74) Mandataire: Goulard, Sophie

(56) Documents cités:
- EP-A- 0 125 093
- EP-A- 0 126 558
- EP-A- 0 339 284
- BE-A- 876 201
- DE-A- 2 300 084
- DE-A- 3 632 737
- US-A- 5 723 664
- CHEMICAL ABSTRACTS, vol. 72, no. 13, 1970, Columbus, Ohio, US; abstract no.: 66338, S.P. VON HALASZ ET AL.: "prep of new aminosulfur monofluoride imides and aminosulfuroxide monofluoride imides" XP002089526 & CHEM. BER., vol. 103, no. 2, 1970, pages 594-602,

## Description

### DOMAINE DE L'INVENTION

Le domaine de invention est celui de nouveaux solvants polaires et de nouvelles compositions électrolytiques en découlant et possédant un domaine de stabilité élevé, tel que requis pour les applications dans le domaine électrochimique.

### ART ANTÉRIEUR

On connaît les solvants aprotiques polaires comme les carbonates cycliques ou linéaires, les éthers employés seuls ou en mélange, dans de nombreuses compositions électrolytiques. La stabilité de ces composés face à des potentiels très négatifs, proches de ceux des métaux alcalins, ou très positifs (≥ 4V par rapport à Li⁺ / Li°), est loin d'être satisfaisante, et les batteries au lithium comprenant des électrolytes obtenus par dissolution d'un sel de lithium dans ces solvants posent de sérieux problèmes de sécurité. Les composés de types amide linéaire ou cyclique comme la diméthylformamide ou la N-méthylpyrrolidinone possèdent d'excellentes propriétés en tant que solvants mais s'oxydent à des potentiels encore bas, de l'ordre de 3,7 V par rapport à Li⁺ / Li°.

De nombreux matériaux d'électrodes positives, tels que les oxydes mixtes de métaux de transition et de lithium fonctionnent à des potentiels proches de 4 V par rapport à Li⁺ / Li° et nécessitent donc des stabilités d'électrolytes nettement supérieures à cette valeur. Par exemple, les composés Li_{1-y}Co₁₋ₓ₋₂NiₓAl_{y}O₂ dans lesquels x + y ≤ 1 et z ≤ 0,3); les spinelles de manganèse Li_{1-α}Mn₂₋ₓMₓO₄• Li_{1-α}Co_{1-x-y}NiₓAl_{y} dans lesquels 0 ≤ x + y ≤ 1 ; 0 ≤ y ≤ 0,3; 0 ≤ α ≤ 1 et M = Li, Mg, Al, Cr, Ni, Co, Cu, Ni, Fe.

Les brevets US 4,851,307 et US 5,063,124 décrivent une famille d'électrolytes mettant en oeuvre un sel, un polymère solvatant et un solvant aprotique de la famille des sulfamides de formule générale 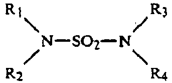 où R¹, R², R³ et R⁴, identiques ou différents, sont choisis indépendamment parmi les groupes C₁₋₁₀alkyles ou C₁₋₁₀oxaalkyles. Un exemple représentatif de ce groupe est la tétraéthylsulfamide (R¹ = R² = R³ = R⁴ = C₂H₅). Ces matériaux ont une stabilité accrue face à des agents réducteurs ou basiques présents ayant des potentiels proches de ceux des métaux alcalins. Ils sont par contre oxydables à des potentiels compris entre 3,8 et 4V par rapport à Li⁺ / Li°.

Les composés décrits dans le brevet européen EP 0 339 284 consistent en des composés diélectriques et isolants constitués par des perfluoro-acylamides ou perfluoro-sulfonamides R_{F}CONA¹A² et R_{F}SO₂NA¹A², où A¹ et A² sont des groupements alkyles. L'utilisation proposée de ces composés dans des condensateurs implique que ces matériaux n'ont pas de conductivité et que les impuretés et contaminations inéluctables, en particulier par des composés ioniques, n'induit pas d'augmentation de conductivité appréciable.

EP-0 858 94 A1 décrit un composé utilisable dans une composition électrolytique ou comme solvant aprotique, ledit composé répondant à la formule R₁R₂NSO₂NR₃R₃ dans laquelle un à trois radicaux parmi R¹, R², R³ et R⁴ sont un groupe méthyle, le ou les autres étant des groupes éthyle, ou un radical parmi R¹, R², R³ et R⁴ est un groupe méthoxyéthyle, les autres radicaux étant des groupes méthyle ou éthyle.

La publication de Sartori et al. dans un abrégé d'une rencontre de l'Electrochemical Society, Volume 97-1, mai 1997, décrit entre autres, que certains sulphonamides pourraient être utilisés comme électrolyte dans une batterie ou dans un système de stockage d'énergie.

### SOMMAIRE DE L'INVENTION

La présente invention concerne une série de nouveaux solvants polaires et de nouvelles compositions électrolytiques en découlant et possédant un domaine de stabilité élevé, tel que requis pour les applications dans le domaine électrochimique. Plus spécifiquement, les solvants de la présente invention correspondent à la formule générale

R¹R²NX(Z)R⁷

dans laquelle
X = C ou SO;
Z = O, NSO₂NR₃R₄ ou NCN;
R¹ et R² sont identiques ou différents et représentent C₁₋₁₈alkyle, C₁₋₁₈ oxaalkyle, C₁₋₁₈ alkylène ou C₁₋₁₈oxaalkylène;
R³ et R⁴ sont identiques ou différents et représentent C₁₋₁₈alkyle ou C₁₋₁₈ oxaalkyle; R⁷ est un groupement R_{F}, un groupement R_{F}CH₂O-, (R_{F})₂CHO- ou (R_{F}CH₂)₂N- ;
R_{F} est un groupement C₁₋₄alkyle, C₁₋₄oxaalkyle ou C₁₋₄azaalkyle dans lesquels le groupement alkyle est essentiellement fluoré et préférentiellement partiellement chloré.

L'expression "essentiellement fluoré" signifie que le degré de fluoration de la chaîne est suffisant pour conférer des propriétés semblables à celles des chaînes entièrement perfluorées, telles que le caractère hydrophobe et des propriétés d'attracteur d'électrons. De préférence, au moins environ la moitié des atomes d'hydrogène de la chaîne sont remplacés par des atomes de fluor. L'expression "partiellement chlorés" signifie que parmi les composés partiellement fluorés, les atomes d'hydrogène restant sont au moins partiellement remplacés par des atomes de chlores.

### DESCRIPTION DÉTAILLÉE DE L'INVENTION

Dans la présente invention, des matériaux possédant une fonction amide très polaire sont utilisés comme base pour la préparation de compositions électrolytiques utiles pour les applications électrochimiques. Il a été trouvé que, de façon tout à fait inattendue, des groupements très fortement attracteurs d'électrons associés à la fonction amide permettent de maintenir un pouvoir solubilisant face à des composés ioniques, en particulier ceux dont la charge anionique est fortement délocalisée, et ainsi d'induire des conductivités ioniques élevées. En ajoutant un polymère polaire à ces compositions, on obtient des électrolytes ayant des propriétés mécaniques facilitant leur mis en oeuvre, en particulier sous forme de films, pour leur utilisation dans des dispositifs électrochimiques et augmentant leur sécurité de fonctionnement. Dépendant des quantités respectives de solvant polaire et de polymère dans les compositions électrolytiques, la consistance de celles-ci s'apparente à celle d'un gel ou à celle d'un polymère plastifié. En outre, les polymères peuvent être réticulés pour améliorer les propriétés mécaniques.

Par rapport aux matériaux de l'art antérieur, les nouvelles compositions électrolytiques de la présente invention possèdent une stabilité accrue, en particulier aux potentiels très anodiques, spécialement ceux excédant 4V par rapport à Li⁺ / Li°.

Les sels de faible énergie réticulaires préférentiels qui sont solubles dans les solvants polaires de la présente invention pour former des solutions conductrices incluent ceux ayant une charge délocalisée, tels que I⁻, ClO₄⁻, BF₄⁻, PF₆⁻, AsF₆⁻, SbF₆⁻, R_{F}SO₃⁻, XSO₂NSO₂X'⁻ , (XSO₂)(X'SO₂)(Y)C⁻ et leurs mélanges, dans lesquels
- X et X' sont choisis parmi R_{F}, R_{F}CH₂O- (R_{F})₂CHO-, (R_{F}CH₂)₂N-, R⁸, R⁹R¹⁰N-, avec la restriction qu'au moins un X ou X' est choisi parmi R_{F}, R_{F}CH₂O-, (R_{F})₂CHO-, (R_{F}CH₂)₂N-;
- Y = R_{F}, R_{F}SO₂ ou CN;
- R_{F} est tel que défini précédemment; et
- R⁸ à R¹⁰ sont identiques ou différents, et représentent C₁₋₁₈alkyles ou C₁₋₁₈oxaalkyle; R_{F} et R⁸ -R¹⁰ pouvant faire partie d'une chaîne macromoléculaire. Sont aussi préférés les anions dérivés du 4,5-dicyano-1,2,3-triazole, du 3,5-bis(R_{F})-1,2,4-triazole, le tricyano-méthane, le pentacyanocyclopentadiène et le pentakis(trifluorométhyl)cyclopentadiène et les anions dérivés de la cyanamide et du malononitrile, i.e., R_{F}SO₂NCN⁻, C(CN)₃⁻, R_{F}SO₂C(CN)₂⁻. Les cations préférentiels sont choisis parmi ceux dérivés des métaux alcalins, en particulier le lithium, des métaux alcalino-terreux, les cations organiques de type "onium", en particulier les ammonium, imidazolium, sulfonium, phosphonium et oxonium.

Parmi les compositions électrolytiques faisant l'objet de la présente invention, mentionnons celles contenant au moins un solvant polaire tel que défini précédemment en combinaison avec une ou plusieurs autres molécules polaires en tant que co-solvant. Parmi ces autres molécules polaires, citons les solvants susceptibles de former des mélanges compatibles, par exemple les éthers di-alkyliques de l'éthylène glycol, du diéthylène glycol, du triéthylène glycol, des polyéthylènes glycols ayant préférablement une masse comprise entre 400 et 2000; ou les esters, en particulier ceux de l'acide carbonique, linéaires ou cycliques, tels que le diméthylcarbonate, le méthyl-éthylcarbonate, le diéthylcarbonate, le carbonate d'éthylène, le carbonate de propylène; ou des esters comme la γ-butyrolactone, les nitriles comme le glutaronitrile, ou le 1,2,6-tricyanohexane. Ces autres molécules polaires, ou co-solvant, peuvent être ajoutés seules au solvant de la présente invention, ou en mélange. Un exemple de mélange préférentiel est le mélange du carbonate d'éthylène avec un éther di-alkylique.

La présente invention inclut en outre les électrolytes solides obtenus par addition d'un polymère aux solvants ou mélange de solvants - co-solvants contenant au moins un sel en solution tels que définis ci-dessus. La proportion de polymère peut être choisie de manière à ce que le solvant agisse comme plastifiant du polymère, dans une fraction de 3 à 30% en poids, de préférence de 10 à 25% en poids. Les polymères préférés pour ces compositions sont ceux dont les unités monomères possèdent des unités solvatantes, tels ceux dérivés de l'oxyde d'éthylène, l'oxyde de propylène, l'épichlorohydrine, l'épifluorohydrine, le trifluoroépoxypropane etc. Le polymère peut aussi servir à constituer un gel quand la fraction massique du solvant et du sel est comprise entre 30 et 95% en poids, de préférence de 40 à 70%. Outre les polymères précités, ceux contenant des unités dérivées de l'acrylonitrile, du méthacrylate de méthyle, du fluorure de vinylidène, de la N-vinylpyrolidinone sont également avantageux, et peuvent être des homo- ou des co-polymères. En particulier, on peut citer les copolymères du fluorure de vinylidène et de l'hexafluoropropène. Un copolymère contenant de 5 à 30% molaire d'hexafluoropropène est particulièrement avantageux. Dans une variante, les polymères sont des polyélectrolytes incorporant dans la trame macromoléculaire des anions de type de ceux à charge delocalisée. Dans ces conditions, les charges négatives sont immobilisées et seules les contre-charges positives participent au processus de conduction ionique.

Les compositions électrolytiques de l'invention sont utilisables dans tous les cas où une grande stabilité est requise, plus particulièrement vis-à-vis de l'oxydation ou des potentiels très positifs. Un bon exemple est un générateur électrochimique dans lequel il est avantageux de disposer d'une force électromotrice élevée, et plus particulièrement les générateurs mettant en jeu l'ion lithium. Dans un tel système, l'électrode négative est constituée de lithium métallique, d'un de ses alliages, un composé d'insertion du carbone, en particulier du coke de pétrole ou du graphite, un oxyde à bas potentiel d'insertion tel que les spinelles de titane Li_{2x+1+3y}Tiₓ₊₅O₁₂ (x ≥ 0 et y ≤ 1), un nitrure double d'un métal de transition et de lithium comme Li₃₋ₓCoₓN, ou ayant la structure de type antifluorite comme Li₃FeN₂ ou Li₇MnN₄.

Les matériaux d'électrode positive sont choisis parmi les composés d'insertion, les polydisulfures ou les oxocarbones. Parmi les composés d'insertion, sont préférés l'oxyde de vanadium, et de façon préférentielle celui de formule VOₓ où 2 ≤ x ≤ 2,5; l'oxyde mixte de lithium et de vanadium LiV₃O₈; les oxydes doubles de cobalt et de lithium partiellement substitués de formule générale Li_{1-α}Co₁₋ₓ-NiₓAl_{y} dans lequel 0 ≤ x + y ≤ 1 ; 0 ≤ y ≤ 0,3 ; 0 ≤ α ≤ 1; les spinelles de manganèse partiellement substitués de formule générale Li_{1-α}Mn₂₋₂M₂O₄ dans lequel 0 ≤ z ≤ 1 et M = Li, Mg, Al, Cr, Ni, Co, Cu, Ni, Fe; et les phosphates doubles de structure olivine ou Nasicon tels que Li_{1-α}Fe₁₋ₓMnₓPO₄, Li_{1-α-2x}Fe₂P₁₋ₓSₓO₄, dans lesquels x ≥ 0 et α ≤ 1. Les matériaux d'électrode de type oxocarbones sont de préférence choisis parmi les sels de l'acide rhodizonique, les polydisulfure sont choisis parmi ceux dérivés de l'oxydation du dimercaptoéthane, du 2,5-dimercapto-1,3,4-thiadiazole, du 2,5-dimercapto-1,3,4-oxaadiazole, du 1,2-dimercaptocyclobutène-3,4-dione.

Les générateurs électrochimiques utilisant les compositions électrolytiques selon l'invention utilisent de préférence des électrolytes solides, de type plastifié ou sous forme de gel. Dans une réalisation préférée de l'invention, au moins une des électrodes est sous forme de composite contenant le ou les matériaux d'électrodes mélangés à la composition électrolytique et du carbone sous forme divisée, tel le noir de Shawinigan^{®}, le Ketjenblack^{®}, le graphite.

Une autre application de l'invention est celle des supercapacités, dans lesquelles au moins une électrode est constituée de carbone de haute surface spécifique et l'énergie électrique est stockée par la capacité de la double couche entre le matériau carboné et l'électrolyte. Dans un mode de réalisation préférentiel, les deux électrodes sont symétriquement faites à base de carbone de haute surface spécifique, et ce matériau est mis en oeuvre sous forme de composite en mélange avec l'électrolyte. Une autre possibilité consiste à utiliser un matériau d'électrode contenant au moins un polymère possédant des doubles liaisons conjuguées. Dans un mode préféré de réalisation, le polymère conjugué peut présenter trois degrés d'oxydation, obtenus par réduction (dit dopage "n") concomitant à une injection d'électrons et de cations, ou par oxydation (dit dopage "p") concomitant à une extraction d'électrons et injection d'anions, à partir de la forme neutre. Les polymères à base de phényl-3-thiophène, en particulier le poly(4-fluorophényl-3-thiophène) sont particulièrement préférés.

Les exemples fournis ci-dessous le sont afin d'illustrer des modes de réalisations préférentiels de la présente invention, et ne devraient en aucun cas être considérés comme limitant la portée de l'invention.

### Exemple 1

Du trifluoroéthanol (18.2 mL, 25 mml) est dissous dans 100 mL d'éther et cette solution est ajoutée à 7 g d'hydrure de sodium. Lorsque la génération de gaz a cessé, la solution est centrifugée, et le liquide surnageant clair est ajouté à 0°C à 35 µg (25 mml) de chlorure de diméthylsulfamoyle dissous dans 100 mL d'éther sec sous agitation. Un précipité blanc de chlorure de sodium est alors formé et la réaction est complète après 2 heures. La suspension résultante est filtrée et l'éther est évaporé à l'aide d'un évaporateur rotatif. Le résidu est placé dans 50 ml de dichlorométhane et lavé avec une solution aqueuse 10% d'acide chlorhydrique. La phase organique est ensuite séparée et séchée avec du sulphate de magnésium anhydre. Le produit résultant, le N,N diméthylsulfamate de trifluoéthyle est distillé sous pression réduite. RMN: ¹⁹F: triplet δ = 74.7 ppm, *J_{HF}* = 8.1 Hz; ¹H: quadruplet δ = 4.66 (2H), singulet δ = 3.6 (6H). La conductivité des sels de lithium de la bis(trifluorométhanesulfonimide) (CF₃SO₂)₂NLi en solution dans ce solvant est donnée en fonction de la concentration dans le Tableau 1.

**Tableau 1**

| molalité (mol.kg⁻¹) | conductivité κₛₚ (S.cm⁻¹) |
|---|---|
| 0.265 | 0.634 |
| 0.506 | 0.922 |
| 0.898 | 1.025 |
| 1.160 | 0.796 |

Le domaine de stabilité électrochimique a été mesuré par voltammétrie cyclique sur microélectrode de platine de 15 µm de diamètre pour l'exploration des potentiels anodiques, de nickel pour les potentiels cathodiques. Le domaine de stabilité est de 0 à 5.2 V vs. Li+ / Li°. La variation de la conductivité en fonction de la température est donnée dans le Tableau 2 suivant pour une concentration de 0.898 mol.kg⁻1.

**Tableau 2**

| T(°C) | κₛₚ (S.cm⁻¹) | T (°C) | κₛₚ (S.cm⁻¹) |
|---|---|---|---|
| 14.90 | 0.753 | 30.29 | 1.203 |
| 14.92 | 0.7550 | 35.39 | 1.415 |
| 19.93 | 0.882 | 40.81 | 1.657 |
| 25.11 | 1.030 | | |

### Exemple 2

À 6.3 mL de 1,1,1,-3,3,3,-hexafluoropropanol dans 25 mL l'éther anhydre sont ajoutés 1.6 g d'hydrure de sodium. Lorsque le dégagement d'hydrogène a cessé, la solution est centrifugée, et au liquide surnageant sont ajoutés 8.6 µg (60 mml) de chlorure de diméthylsulfamoyle dissous dans 25 mL d'éther sec à 0°C sous agitation. Un précipité blanc de chlorure de sodium est alors formé et la réaction est complète après 2 heures. La suspension résultante est filtrée et l'éther est évaporé à l'aide d'un évaporateur rotatif. Le résidu est placé dans 20 mL de dichlorométhane et lavé avec une solution aqueuse 10% d'acide chlorhydrique. La phase organique est ensuite séparée et séchée avec du sulphate de magnésium anhydre. Le produit résultant, le N,N-diméthylsulfamate d'hexafluoropropyle, est obtenu par évaporation du dichlorométhane et purifié par distillation sous pression réduite. Sa constante diélectrique est supérieure à 20, et les solutions de sels de bis(trifluorométhanesulfonimide) (NC₂H₅)₄(CF₃SO₂)₂N en solution dans ce solvant est comprise entre 5 x 10⁻⁴ et 2 x 10⁻³ Scm⁻¹ à 25°C dans la gamme de concentration 0,2 à 1 mole.kg⁻¹.

### Exemple 3

Un générateur électrochimique constitué d'une électrode négative de lithium de 25 µm sur support de nickel de 10 µm, d'une électrode positive composite contenant 78 % en poids d'oxyde de vanadium V₂O₅, 8% de noir de carbone (Ketjenblack®) et 14% de copolymère de fluorure de vinylidène et d'hexafluoropropène sur un collecteur de nickel (10 µm) a été réalisé. La capacité de l'électrode positive ainsi obtenue par épandage à partir d'une suspension dans du cyclohexanone est de 2.8 mAh/cm². L'électrolyte est constitué par une solution de 0.15 M.kg⁻¹ de Li(CF₃SO₂)₂N dans le composé polaire de l'exemple 1 dans un séparateur poreux en polypropylène de type Celgard^{®}. Le générateur a été cyclé sur 150 cycles entre 1.6 et 3.4V à C/3.7 en maintenant un rapport des capacités de charge et de décharge égal à 1 et un taux d'utilisation > 75% sur 30 cycles. La chute ohmique est restée comprise entre 20 et 120 mV.

### Exemple 4

Un générateur électrochimique de type "rocking chair" a été construit avec deux électrodes composites similaires à celles de l'Exemple 3. Le matériau de l'électrode négative est le spinelle de lithium et de titane Li₄Ti₅O₁₂ pour une capacité surfacique de 2.6 mAh.cm⁻². Le matériau de l'électrode positive est le cobaltite de lithium pour une capacité surfacique de 2.4 mAh.cm⁻². L'électrolyte est constitué d'une manière similaire à celui de l'Exemple 6 par une solution de 0,15 M.kg⁻¹ de Li(CF₃SO₂)₂N dans le composé polaire de l'Exemple 1 dans un séparateur poreux en polypropylène de type Celgard^{®}. Le générateur a été cyclé sur 500 cycles entre 1,5 et 3,3 V à C/4 en maintenant un rapport des capacités de charge et de décharge égal à 1 et un taux d'utilisation de 80%.

### Exemple 5

Un générateur électrochimiques de type supercapacité a été construit avec deux électrodes composites symétriques de carbone de haute surface spécifique (680 m².g⁻¹) et de fibres de nickel sur un support de nickel et liées par un copolymère de fluorure de vinylidène et d'hexafluoropropène. L'électrolyte est constitué par un gel à 75% en poids d'une solution molaire de fluorosulfonimidure de tétraéthylammonium (C₂H₅)₄N[(CF₃SO₂)₂N] dans le même polymère. La capacité du système ainsi construit est de 1.2 F.g⁻¹ sur 12000 cycles effectuées entre 0 et 2.5 V.

Bien que la présente invention ait été décrite à l'aide de mises en oeuvre spécifiques, il est entendu que plusieurs variations et modifications peuvent se greffer aux dites mises en oeuvre, et la présente demande vise à couvrir de telles modifications, usages ou adaptations de la présente invention suivant, en général, les principes de l'invention et incluant toute variation de la présente description qui deviendra connue ou conventionnelle dans le champ d'activité dans lequel se retrouve la présente invention, et qui peut s'appliquer aux éléments essentiels mentionnés ci-haut, en accord avec la portée des suivantes.

## Revendications

1. Composé aprotique polaire ayant des propriétés de solvant et répondant à la formule générale :
R¹R²NX(Z)R⁷
dans laquelle
X=C ou SO;
Z = O, NSO₂NR³R⁴ ou NCN;
R¹ et R² sont identiques ou différents et représentent C₁₋₁₈alkyle, C₁₋₁₈ oxaalkyle, C₁₋₁₈ alkylène ou C₁-₁₈oxaalkylène;
R³ et R⁴ sont identiques ou différents et représentent C₁₋₁₈alkyle ou C₁₋₁₈ oxaalkyle;
R⁷ est un groupement R_{F}, un groupement R_{F}CH₂O-, (R_{F})₂CHO-, ou (R_{F}CH₂)₂N- ;
R_{F} est un groupement C₁₋₄alkyle, C₁₋₄oxaalkyle ou C₁₋₄azaalkyle, ledit groupement étant essentiellement fluoré.

2. Composition électrolytique comprenant au moins un composé polaire selon la revendication 1, un sel soluble dans ledit composé et ayant un anion à charge délocalisée, et au moins un polymère.

3. Composition électrolytique selon la revendication 2 dans laquelle le sel inclut I⁻, ClO₄⁻, BF₄⁻, PF₆⁻, AsF₆⁻, SbF₆⁻, R_{F}SO₃⁻, XSO₂NSO₂X'⁻, (XSO₂)(X'SO₂)(Y)C⁻, les anions dérivés du 4,5-dicyano-1,2,3-triazole, du 3,5-bis(R_{F})-1,2,4-triazole, le tricyano-méthane, le pentacyanocyclopentadiène et le pentakis(trifluorométhyl)cyclopentadiène, R_{F}SO₂NCN⁻, C(CN)₃⁻, R_{F}SO₂C(CN)₂⁻, et leurs mélanges, dans lesquels,
- X et X' sont choisis parmi R_{F}, R_{F}CH₂O-, (R_{F})₂CHO-, (R_{F}CH₂)₂N-, R⁸, R⁹R¹⁰N-, avec la restriction qu'au moins un X ou X' est R_{F}, R_{F}CH₂O-, (R_{F})₂CHO-, ou (R_{F}CH₂)₂N-;
- Y = R_{F}, R_{F}SO₂ ou CN;
- R_{F} est tel que défini précédemment et peut faire partie d'une chaîne macromoléculaire; et
- R⁸ à R¹⁰ sont identiques ou différents, et représentent C₁₋₁₈alkyles ou C₁₋₁₈ oxaalkyle, et R⁸ -R¹⁰ peuvent faire partie d'une chaîne macromoléculaire.

4. Composition électrolytique selon la revendication 3, **caractérisée en ce que** le cation inclut ceux dérivés des métaux alcalins, des métaux alcalino-terreux, les cations organiques de type "onium", en particulier les ammonium, imidazolium, sulfonium, phosphonium, oxonium et leurs mélanges.

5. Composition électrolytique selon la revendication 4, **caractérisée en ce que** le cation est au moins en partie du lithium.

6. Composition électrolytique selon la revendication 2, **caractérisée en ce qu'**elle comprend, en plus du composé polaire, un co-solvant.

7. Composition électrolytique selon la revendication 6, **caractérisée en ce que** le co-solvant est aprotique et polaire, et inclut les éthers di-alkyliques de l'éthylène glycol, du diéthylène glycol, du triéthylène glycol, des polyéthylène glycols ; les esters, en particulier ceux de l'acide carbonique, linéaires ou cyclique tels le diméthylcarbonate, le méthyl-éthylcarbonate, le diéthylcarbonate, le carbonate d'éthylène, le carbonate de propylène ; les esters comme la γ-butyrolactone, les nitriles comme le glutaronitrile, le 1,2,6-tricyanohexane ainsi que les mélanges des composés précités, et les amides comme le diméthyl formamide, la N-méthylpyrrolidinone.

8. Composition électrolytique selon la revendication 7, **caractérisée en ce que** la masse du polyéthylène glycol est comprise entre 400 et 2000.

9. Composition électrolytique selon la revendication 2, **caractérisée en ce que** le polymère inclut ceux dont les unités monomères sont dérivées de l'oxyde d'éthylène, l'oxyde de propylène, l'épichlorohydrine, l'épifluorohydrine, le trifluoroépoxypropane, l'acrylonitrile, le méthacrylate de méthyle, le fluorure de vinylidène, la N-vinylpyrolidinone, l'hexafluoropropène, chacun sous forme d'homo- ou de co-polymère, et leurs mélanges.

10. Composition électrolytique selon la revendication 7, **caractérisée en ce qu'**elle est plastifiée ou sous forme de gel.

11. Composition électrolytique selon la revendication 8, **caractérisée en ce qu'**au moins un des polymères est un polyélectrolyte incorporant dans la trame macromoléculaire des anions de type de ceux à charge délocalisée.

12. Générateur électrochimique **caractérisé en ce qu'**il utilise comme électrolyte une composition électrolytique selon la revendication 2.

13. Générateur électrochimique selon la revendication 12, **caractérisé en ce que** l'électrode négative contient du lithium métallique, un de ses alliages, un composé d'insertion du carbone, un oxyde à bas potentiel d'insertion, un nitrure double d'un métal de transition et de lithium, ou leurs mélanges.

14. Générateur électrochimique selon la revendication 13 dans lequel le composé d'insertion du carbone est du coke de pétrole ou du graphite, et dans lequel l'oxyde à bas potentiel d'insertion est un spinelle de titane.

15. Générateur électrochimique selon la revendication 13, **caractérisé en ce que** l'électrode positive contient de l'oxyde de vanadium, de l'oxyde mixte de lithium et de vanadium, un oxyde double de cobalt et de lithium, un spinelle de manganèse; un phosphate double de structure olivine ou Nasicon, un sel de l'acide rhodizonique, un polydisulfure dérivé de l'oxydation du dimercaptoéthane, du 2,5-dimercapto-1,3,4-thiadiazole, 2,5-dimercapto-1,3,4-oxadiazole, du 1,2-dimercaptocyclobutène-3,4-dione ou leurs mélanges.

16. Système de stockage de l'énergie électrique de type supercapacité, **caractérisé en ce qu'**il utilise comme électrolyte une composition électrolytique selon la revendication 2.

## Patentansprüche

1. Polare aprotische Verbindung mit Lösungsmitteleigenschaften, die der allgemeinen Formel entspricht:
R¹R²NX(Z)R⁷
wobei:
X=C oder SO,
Z = O, NSO₂NR₃R₄ oder NCN,
R¹ und R² identisch oder unterschiedlich sind und C₁₋₁₈-Alkyl, C₁₋₁₈-Oxaalkyle, C₁₋₁₈-Alkylen oder C₁₋₁₈-Oxaalkylen darstellen,
R³ bis R⁶ identisch oder unterschiedlich sind und C₁₋₁₈-Alkyl oder C₁₋₁₈-Oxaalkyl dartstellen,
R⁷ eine Gruppe R_{F}, eine Gruppe R_{F}CH₂O-, (R_{F})₂CHO- oder (R_{F}CH₂)₂N- ist,
R_{F} eine Gruppe C₁₋₄-Alkyl, C₁₋₄-Oxaalkyl oder C₁₋₄-Azaalkyl ist, wobei die Gruppe im Wesentlichen fluoriert ist.

2. Elektrolytische Zusammensetzung, umfassend wenigstens eine polare Verbindung nach Anspruch 1, wobei ein lösliches Salz in der Verbindung und mit einem Anion mit delokalisierter Ladung und wenigstens ein Polymer.

3. Elektrolytische Zusammensetzung nach Anspruch 2, wobei das Salz I⁻, ClO4⁻, BF₄⁻, PF₆⁻, AsF₆⁻, SbF6⁻, R_{F}SO₃⁻, XSO₂NSO₂X'⁻, (XSO₂)(X'SO₂)(Y)C⁻, die Derivatanionen des 4,5-Dicyan-1,2,3-triazols, des 3,5-bis(R_{F})-1,2,4-triazols, Tricyanmethan, Pentacyancyclopentadien und Pentakis(trifluormethyl)cyclopentadien, R_{F}SO₂NCN⁻, C(CN)₃⁻ , R_{F}SO₂C(CN)₂⁻ und deren Gemische einschließt, wobei
- X und X' aus R_{F}, R_{F}CH₂O-, (R_{F})₂CHO-, (R_{F}CH₂)₂N-, R⁸, R⁹R¹⁰N- ausgewählt sind, mit der Einschränkung, dass wenigstens ein X oder X' R_{F}, R_{F}CH₂O-, (R_{F}) ₂CHO- oder (R_{F}CH₂)₂N- ist,
- Y = R_{F}, R_{F}SO₂ oder CN,
- R_{F} wie zuvor definiert ist und Teil einer makromolekularen Ketten sein kann und
- R⁸ bis R¹⁰ identisch oder unterschiedlich sind und C₁₋₁₈-Alkyle oder C₁₋₁₈-Oxaalkyl darstellten, und R⁸-R¹⁰ Teil ener makromolekularen Kette sein können.

4. Elektrolytische Zusammensetzung nach Anspruch 3, **dadurch gekennzeichnet, dass** das Kation diejenigen einschließt, die Derivate der alkalischen Metalle, der erdalkalischen Metalle, die organischen Kationen vom Typ "Onium", insbesondere die Ammonium, Imidazolium, Sulfonium, Phosphonium, Oxonium und deren Gemische sind.

5. Elektrolytische Zusammensetzung nach Anspruch 4, **dadurch gekennzeichnet, dass** das Kation wenigstens zum Teil Lithium ist.

6. Elektrolytische Zusammensetzung nach Anspruch 2, **dadurch gekennzeichnet, dass** sie zusätzlich zur polaren Verbindung ein Co-Solvens umfasst.

7. Elektrolytische Zusammensetzung nach Anspruch 6, **dadurch gekennzeichnet, dass** das Co-Solvens polar und aprotisch ist und die Dialkylether des Ethylenglucols, des Diethylenglycols, des Triethylenglycols, der Polyethyleneglycole, die Ester, insbesondere die der Carbonsäure, linear oder zyklisch, wie das Dimethylcarbonat, das Methylethylcarbonat, das Diethylcarbonat, das Ethylencarbonat, das Propylencarbonat, die Ester wie das γ-Butyrolacton, die Nitrile wie das Glutaronitril, das 1,2,6-Tricyanohexan sowie die Gemische der vorgenannten Verbindung und die Amide wie das Dimethylformamid, das N-methylpyrrolidinon einschließt.

8. Elektrolytische Zusammensetzung nach Anspruch 7, **dadurch gekennzeichnet, dass** die Masse des Polyethylenglycols zwischen 400 und 2000 inklusive ist.

9. Elektrolytische Zusammensetzung nach Anspruch 2, **dadurch gekennzeichnet, dass** das Polymer diejenigen einschließt, deren Monomereinheiten Derivate vom Ethylenoxid, Propylenoxid, Epichlorhydrin, Epifluorhydrin, Trifluorepoxypropan, Acrylnitril, Methylmethacrylat, Vinylidenfluorid, N-vinylpyrolidinon, Hexafluorpropen, jeweils in ihrer Homo- oder Co-Polymer-Form, und deren Gemische sind.

10. Elektrolytische Zusammensetzung nach Anspruch 7, **dadurch gekennzeichnet, dass** sie plastiziert oder in Gelform ist.

11. Elektrolytische Zusammensetzung nach Anspruch 8, **dadurch gekennzeichnet, dass** wenigstens eines der Polymere ein Polyelektrolyt ist, das in der makromolekularen Kette Anionen vom Typ derjenigen mit delokalisierter Ladung einschließt.

12. Elektrochemischer Generator, **dadurch gekennzeichnet, dass** er als Elektrolyt eine elektrolytische Zusammensetzung nach Anspruch 2 verwendet.

13. Elektrochemischer Generator nach Anspruch 12, **dadurch gekennzeichnet, dass** die negative Elektrode ein metallisches Lithium, eine seiner Legierungen, eine Kohlenstoffinsertionsverbindung, ein Oxid mit niedrigem Insertionspotential, ein Doppelnitrid eines Übergangsmetalls und von Lithium oder deren Gemische enthält.

14. Elektrochemischer Generator nach Anspruch 13, wobei die Kohlenstoffinsertionsverbindung Petrolkoks oder Graphit ist und wobei das Oxid mit niedrigem Insertionspotential ein Titanspinell ist.

15. Elektrochemischer Generator nach Anspruch 13, **dadurch gekennzeichnet, dass** die positive Elektrode Vanadiumoxid, Lithium- und Vanadiummischoxid, ein Kobalt- und Lithiumdoppeloxid, einen Manganspinell; ein Doppelphosphat mit Olivinstruktur oder Nasicon, ein Salz der Rhodizonsäure, ein Polydisulfid als Derivat der Oxidation des Dimercaptoethans, des 2,5-Dimercapto-1,3,4-thiadiazols, 2,5-Dimercapto-1,3,4-oxadiazols, des 1,2-Dimercaptocyclobuten-3,4-dions oder deren Gemische enthält.

16. Speichersystem elektrischer Energie vom Typ Superkapazität, **dadurch gekennzeichnet, dass** es als Elektrolyt eine elektrolytische Zusammensetzung nach Anspruch 2 verwendet.

## Claims

1. An aprotic polar compound having solvent properties and having general formula:
R¹R²NX(Z)R⁷
wherein
X = C or SO;
Z = O, NSO₂NR³R⁴ or NCN;
R¹ and R² are identical or different and represent C₁₋₁₈ alkyl, C₁₋₁₈ oxaalkyl, C₁₋₁₈ alkylene or C₁₋₁₈ oxaalkylene;
R³ and R⁴ are identical or different and represent C₁₋₁₈ alkyl or C₁₋₁₈ oxaalkyl;
R⁷ is an R_{F} group, a R_{F}CH_{2O}-, (R_{F})₂CHO-, or (R_{F}CH₂)₂N- group;
R_{F} is a C₁₋₄ alkyl, C₁₋₄ oxaalkyl or C₁₋₄ azaalkyl group, said group being essentially fluorinated.

2. An electrolytic composition comprising at least one polar compound according to claim 1, a salt soluble in said compound and having an anion with a delocalized charge, and at least one polymer.

3. The electrolytic composition according to claim 2, wherein the salt includes I⁻, ClO₄⁻, BF₄⁻, PF₆⁻, AsF₆⁻, SbF₆⁻, R_{F}SO₃⁻, XSO₂NSO₂X'⁻, (XSO₂)(X'SO₂)(Y)C⁻, anions derived from 4,5-dicyano-1,2,3-triazole, 3,5-bis(R_{F})-1,2,4-triazole, tricyano-methane, pentacyanocyclopentadiene and pentakis(trifluoromethyl)cyclopentadiene, R_{F}SO₂NCN⁻, C(CN)₃⁻, R_{F}SO₂C(CN)₂⁻, and their mixtures, wherein
- X and X' are chosen from among R_{F}, R_{F}CH₂O-, (R_{F})₂CHO-, (R_{F}CH₂)₂N-, R⁸, R⁹R¹⁰N-, with the restriction that at least one X or X' is R_{F}, R_{F}CH₂O-, (R_{F})₂CHO-, or (R_{F}CH₂)₂N-;
- Y = R_{F}, R_{F}SO₂ or CN;
- R_{F} is as previously defined and may be part of a macromolecular chain; and
- R⁸ to R¹⁰ are identical or different, and represent C₁₋₁₈ alkyl or C₁₋₁₈ oxaalkyl, and R⁸-R¹⁰

4. The electrolytic composition according to claim 3, **characterized in that** the cation includes those derived from alkali metals, alkaline earth metals, organic cations of the "onium" type, in particular ammonium, imidazolium, sulfonium, phosphonium, oxonium and mixtures thereof.

5. The electrolytic composition according to claim 4, **characterized in that** the cation is at least partially lithium.

6. The electrolytic composition according to claim 2, **characterized in that** in addition to the polar compound, it comprises a co-solvent.

7. The electrolytic composition according to claim 6, **characterized in that** the cosolvent is aprotic and polar, and includes the dialkyl ethers of ethylene glycol, diethylene glycol, triethylene glycol, polyethylene glycols; esters, in particular those of carbonic acid, linear or cyclic, such as dimethyl carbonate, ethyl methyl carbonate, diethyl carbonate, ethylene carbonate, propylene carbonate; esters such as γ-butyrolactone, nitriles such as glutaronitrile, 1,2,6-tricyanohexane as well as mixtures of the aforementioned compounds, and amides, such as dimethylformamide, N-methylpyrrolidinone.

8. The electrolytic composition according to claim 7, **characterized in that** the mass of the polyethylene glycol is comprised between 400 and 2,000.

9. The electrolytic composition according to claim 2, **characterized in that** the polymer includes those whereof the monomer units are derived from ethylene oxide, propylene oxide, epichlorohydrine, epifluorohydrine, trifluoroepoxypropane, acrylonitrile, methyl methacrylate, vinylidene fluoride, N-vinylpyrolidinone, hexafluoropropene, each in homo- or co-polymer form, and mixtures thereof.

10. The electrolytic composition according to claim 7, **characterized in that** it is plasticized or in gel form.

11. The electrolytic composition according to claim 8, **characterized in that** at least one of the polymers is a polyelectrolyte incorporating anions of the type having a delocalized charge into the macromolecular frame.

12. An electrochemical generator, **characterized in that** as electrolyte, it uses an electrolytic composition according to claim 2.

13. The electrochemical generator according to claim 12, **characterized in that** the negative electrode contains metal lithium, one of its alloys, a carbon insertion compound, an oxide with a low insertion potential, a double nitride of a transition metal and lithium, or mixtures thereof.

14. The electrochemical generator according to claim 13, wherein the carbon insertion compound is petroleum coke or graphite, and wherein the oxide with low insertion potential is a titanium spinel.

15. The electrochemical generator according to claim 13, **characterized in that** the positive electrode contains vanadium oxide, mixed lithium and vanadium oxide, a double oxide of cobalt and lithium, a manganese spinel; a double phosphate with an olivine or Nasicon structure, a salt of rhodizonic acid, a polydisulfide derived from the oxidation of dimercaptoethane, 2,5-dimercapto-1,3,4-thiadiazole, 2,5-dimercapto-1,3,4-oxadiazole, 1,2-dimercaptocyclobutene-3,4-dione or mixtures thereof.

16. An electricity storage system of the supercapacitor type, **characterized in that** as electrolyte, it uses an electrolytic composition according to claim 2.
